# EUROPEAN PATENT APPLICATION

(11) **EP 4 361 326 A1**
(43) Date of publication of application: **01.05.2024**
(21) Application number: 22383048.0
(22) Date of filing: 31.10.2022
(51) Int. Cl.: D01F 4/02, D01D 5/06, D01F 1/10, D01F 1/02, A61L 27/36

(54) **METHOD FOR GENERATING A PLURALITY OF BIOCOMPATIBLE FIBRES**

(71) Applicant: Universidad de Sevilla, 41013 Sevilla (ES)
(72) Inventor: GAÑÁN CALVO, Alfonso Miguel, 41013 Sevilla (ES); BLANCO TREJO, Sergio, 41013 Sevilla (ES); MODESTO LÓPEZ, Luis Balam, 41013 Sevilla (ES); GAÑÁN RIESCO, Braulio, 41900 Camas (Sevilla) (ES); GUINEA TORTUERO, Gustavo, 28040 Madrid (ES); PÉREZ RIGUEIRO, José, 28040 Madrid (ES)
(74) Representative: TRBL Intellectual Property

(57) **Abstract**

The present invention discloses a method for generating a plurality of biocompatible fibroin fibres (6) generated from the turbulent mixing produced in the inner mixing region of a nebulizer, among a dope stream comprising a dope solution (2) that comprises fibroin, and a co-flow comprising a coagulating solution (4), producing a mixing compound. Before said mixing, the dope solution (2) is received in an inlet section of the nebulizer at an initial concentration comprised between 5% wt and 50% wt. Likewise, the Schmidt number (Sc) of the co-flow in the inner mixing region of the nebulizer is between 0.5 and 10, and the Reynolds number (Re) of the co-flow in the said inner mixing region is between 1000 and 5000, so that the mixing compound splits into a sequence of fibrous structures in the inner mixing region and a turbulent jet (5) comprising a plurality of fibroin fibres (6) exits the outlet section of the nebulizer.

## Description

### FIELD OF THE INVENTION

The present invention belongs to the field of biomechanical engineering. More specifically, the invention refers to the generation of a plurality biocompatible structures such as fibres, that may be used, for example, as scaffolding in Tissular Engineering, by taking advantage of application of turbulent hydrodynamic conditions to a dope solution comprising fibroin.

### BACKGROUND OF THE INVENTION

Silk spinning organisms, such as silkworms and spiders, have developed an optimised system for the conversion of a highly concentrated protein solution into high-performance solid fibres. The whole spinning mechanism is supported by the peculiar features of the silk proteins (fibroins), that undergo a self-assembly process in the silk glands that results from chemical gradients inside the gland and by the application of mechanical stresses during the last step of the process. This sophisticated process allows producing natural fibres under minimum energy requirements, and, in addition, the properties of the material can be adapted to the immediate necessities of the spinning organism.

The obtention of this kind of fibres at an industrial level can be enormously beneficial for a plethora of applications, especially those related to Tissular Engineering: they could be used as base materials in biomedical composites, hypoallergenic fabrics or pads, as substrates in Controlled Drug Delivery, or as highly resistant raw material for fabrics and films.

For this reason, fabricating solid, resistant fibres is a desirable objective of the market nowadays, and, in this sense, first approaches have been developed by trying to mimic the known, efficient, natural process performed by silkworms and spiders described above. However, the biomimetic developments based on natural spinning must face the enormous demands implied by massively scaling the natural yields found in Nature.

In Nature, the spinning of silk fibres represents a paramount example of conversion of a macromolecular solution into a solid - high performance - material. The whole spinning process is underpinned by a series of singular features that may be identified at several levels, starting from the organization of the genome, which include protein expression, to the physiological mechanisms that finally lead to the assembly of the fibre.

Silk fibres are constituted by a family of proteins called fibroins (or spidroins if just the proteins expressed by spiders are considered). All fibroins share a common design in which a central region, with a large number of repetitions of a few short motifs of sequence, is flanked by specific motifs at the N- and C-termini. The N- and C-termini are highly conserved along evolution and play an essential role in the initial steps of the self-assembly process undergone by the proteins in the silk gland. Subsequently to the initial assembly of the proteins in the gland, the completion of the spinning process requires the formation of β-nanocrystals that result from the piling up of β-pleated sheets both in silkworm and spider spidroins. The β-nanocrystals are formed by motifs that appear in the central repetitive region of the fibroins which include the -GAGAGS- motif in the silkworm Bombyx mori (B. mori) silk and the polyalanine (-An-) motif in major ampullate gland silk.

The formation of the β-nanocrystals is controlled by mechanical stresses exerted on the liquid phase constituted by the self-assembled fibroin proteins, which allow the adaptation of the material to the immediate requirements of the spinning organism. The rheology of the dope solutions, in combination with the geometry of the silk gland and the measured spinning speeds, imply that the natural spinning process proceeds under the conditions of laminar flow, where stresses of approximately 1 MPa are estimated to be required for the formation of the nanocrystals. The application of these stresses takes place while the protein solution is homogeneously elongated through the animal's dragline spinneret, the pH is modulated to provide the adequate hydrogen bonds that facilitate the formation of the crystals, and water is extracted from the solution. Thus, the relatively large stresses of 1 MPa imply a complex but gentle process where the fundamental energy per unit volume is provided by the external pull on the outer fibre created by the animal movements. The animal's internal organs gently respond to the external material and mechanical demands towards an evolutionary optimum in the mechanical performance of the fibre.

Following this rationale, essentially all proposed spinning processes which intend to produce fibres from fibroin solutions are designed to follow a laminar flow regime (see, for instance, Liivak, O., Blye, A., Shah, N. & Jelinski, L. A microfabricated wet spinning apparatus to spin fibers of silk proteins. structure-property correlations. Macromolecules 31 (9), 2947-2951 (1998); Um, I. et al. Wet spinning of silk polymer: I. effect of coagulation conditions on the morphological feature of filament. Int. J. Biol. Macromol. 34 (1-2), 89-105 (2004) and Zhang, F. et al. Regeneration of high-quality silk fibroin fiber by wet spinning from cacl2-formic acid solvent. Acta Biomater. 12 (1), 139-145 (2015)). However, the methods disclosed therein are mono-fibre geniting processes, so that they cannot account for the massive production of fibroin fibres.

Other works have been developed in the same or in a similar direction, for example, in patent US 11110148 B2. There, a method to produce a silk fibroin material is disclosed as well as possible uses thereof. Nevertheless, this method involves a large number of steps and many chemical compounds, some of which may be expensive and/or environmentally unfriendly. Patents EP 3450452 B1 and US 9150668 B2 also present inventions related to the obtention of fibroin compounds or structures, the first referring to a chemically modified fibroin protein and the second to a fibroin hydrogel. Although complementary, these patents do not share the aim of obtaining resistant biocompatible fibres that may be used as scaffolding in tissular engineering.

The field of biomechanical engineering is therefore lacking an efficient method for massively obtaining robust and resistant biocompatible fibres such that they can be used in multiple applications related to Tissular Engineering.

### SUMMARY OF THE INVENTION

The present invention overcomes the aforementioned needs of the prior art providing a method to massively generate biocompatible fibroin fibres, by subjecting a dope solution comprising fibroin to turbulent hydrodynamical conditions.

In a first aspect, the invention provides a method for generating a plurality of fibroin fibres, the method advantageously comprising the steps of:
providing a nebulizer, the nebulizer having a first inlet section, a second inlet section, an inner mixing region arranged to receive a first flow from the first inlet section and a second flow from the second inlet section, and an outlet section;
receiving a dope stream in the first inlet section of the nebulizer at a first flow rate, the dope stream comprising a dope solution of an initial concentration of fibroin in a solvent which comprises water;
receiving a co-flow in the second inlet section of the nebulizer at a second flow rate the co-flow comprising a coagulating solution wherein the solvent is water, and the solute comprises a mixture of a non-toxic carbon-based acid a non-toxic hydrophilic alcohol and wherein the pH value of said coagulating solution is between 2 and 6;
mixing the dope stream and the co-flow in the inner mixing region of the nebulizer, producing a mixing compound so that simultaneously the pH value of the mixing compound decreases with respect to the pH value of the dope solution by the action of the non-toxic carbon-base acid comprised in the co-flow, water is extracted by the non-toxic hydrophilic alcohol comprised in the co-flow as long as the concentration of water in the mixing compound is lower than the concentration of water in the dope solution, and a strain rate is exerted on the fibroin of the mixing compound by the co-flow; and
exiting through the outlet section of the nebulizer a jet comprising the mixing compound.

Further, in this method the initial concentration of fibroin in the dope solution is comprised between 5% wt and 50% wt, the Schmidt number Sc of the water in the dope stream is between 1 and 10000, and the Reynolds number Re of the co-flow in the inner mixing region of the nebulizer is between 100 and 50000, so that the mixing compound splits in the inner mixing region of the nebulizer into a sequence of fibrous structures and the jet that exits the outlet section of the nebulizer is turbulent and comprises a plurality of fibroin fibres.

The main advantage of said method is that the number of steps is reduced in comparison with the methods of the state of art and, by using common and environmentally friendly solutions, a chaotic sequence of numerous fibroin fibrous structures with mechanical properties in a range where at least a fraction of them are nearly identical to those drawn by spinning organisms is produced, but with a productivity many orders of magnitude larger. This is achieved due to the application of a turbulent flow to the dope solution, defined by specific ranges of the Schmidt and Reynolds numbers, instead of the laminar flow found in the silk glands of the spinning organisms and in prior art methods.

As used herein, the term "fibroin" refers to silk proteins that include, but are not limited to, silkworm silk proteins, spider silk proteins, and genetically engineered silk proteins.

As in silk spinning organisms, the spinning process involves the formation of the β-nanocrystals from a dope solution. The β-nanocrystal formation requires the simultaneous modulation of the dope solution pH value so that adequate hydrogen bounds are provided for said formation, the extraction of the water dissolved in said dope solution, and the application of mechanical stresses in the fibroin of the dope solution.

In the present invention, a local elongation of the dope solution of the dope stream takes place when said dope stream contacts the co-flow in the inner mixing region of the nebulizer, so that the surface per unit volume of the dope solution drastically increases. In this context, the dope solution and the coagulating solution mix in the inner mixing region, producing a two-phase mixing compound and, while the pH of the mixing compound decreases due to the acid of the coagulating solution, water is extracted from the fibroin-rich part of the mixing compound by means of the hydrophilic alcohol of the coagulating solution. Herein, a hydrophilic substance refers to any substance whose mixing with water is an exothermic process, as it is the case of ethanol. Moreover, the acid of the solution comprised in the coagulating co-flow must be carbon-base (e.g., acetic acid), so that fibroin proteins are not affected when both fibroin and coagulating solutions mix in the inner mixing region of the nebulizer. Successful pH modulation and water extraction are achieved with acid-alcohol mixtures whose proportion can be comprised between 0.1 and 90% w/w, and whose concentration when said mixture is dissolved in water would be comprised between 10 and 99.99% w/w.

At the same time, when the above mixing takes place in the inner mixing region, the co-flow exerts mechanical stress in the fibroin of the mixing compound, so that fibroin proteins are subject to local strain rates in said inner mixing region.

In this context, in order to generate a sequence of silk fibres from the dope solution, two main conditions are fulfilled in the inner mixing region:
- there are local strain rates (rate of momentum transfer) of turbulent nature exerted on the fibroin proteins that are larger than the rate of water extraction on the mixing compound, so that fibrous structures can be generated form the fibroins of said mixing compound; and
- the mixing time of the dope stream and the co-flow has the same order of magnitude than the residence time of said dope stream and co-flow between the inner mixing region and the outside region downstream of the exit orifice of the nebulizer, so that there is sufficient time for the hydrophilic alcohol to extract the water.

Concerning the first condition, when the dope stream and the co-flow contact in the inner mixing region, if water extraction is faster than the local stretching of the fibroin proteins, local fibroin structures of globular shape are formed. On the other hand, if the local strain rate dominates, the water extraction merely responds to the local increase in surface area associated to the fibroin stretching, and the fibre-like structures are formed from the fibroins of the mixing compound. Remarkably, said competition only occurs if the initial concentration of fibroin in the dope solution is sufficiently high for water extraction to be controlled by the kinematic processes generated by the mechanical actions on the dope stream.

In this context, fibrous structures can be generated from the dope solution when the Schmidt number Sc of the water in the dope is between 1 and 10000, and when the initial concentration of the fibroin in the dope solution is between 5% wt and 50% wt. Outside the range for the initial concentration, either the concentration is so low that no fibres can form, or the concentration is so high that the dope solution is too viscous for the co-flow to drag the dope solution efficiently. The Schmidt number Sc is the dimensionless quotient between mechanical and chemical diffusions in the dope. This number must be larger than the corresponding Schmidt number Sc_{c} of the water with respect to the co-flow compound, wherein the mechanical diffusion measures the rate of momentum transferred to the liquid bulk associated to velocity gradients, while the chemical diffusion measures the rate of mass transfer of water associated to water concentration gradients in either the dope or the co-flow compounds. Equivalently, the Schmidt number Sc_{c} of the co-flow is given by Sc_{c} = η_{c} / (ρ_{c} D_{c}), wherein η_{c} is the viscosity of the coagulating solution comprised in the co-flow, and D_{c} is the mass diffusivity of the water in the dope with respect to the same coagulating solution.

However, although fibrous structures can be generated from the mixing compound when the initial fibroin concentration and the co-flow Schmidt number Sc_{c} in the inner mixing region are within above ranges, the subsequent large strain rates exerted by the co-flow on the fibroin of said mixing compound lead to a drastic decrease in the residence time of the co-flow and the dope stream in the inner mixing region. This means that axial velocity of the mixing compound is considerably large in said region, so that there may be no time enough for successfully mixing the dope and coagulating solutions. In consequence, there may be no sufficient time for the non-toxic hydrophilic alcohol to extract water from the mixing compound.

Thus, in order to satisfy the aforementioned second requirement, the mixing efficiency required for water extraction needs to be maximized so that it is comparable with the residence time between the inner mixing region and the discharge region just downstream of the exit orifice of the nebulizer. Advantageously, in the present invention this is achieved through the implementation of the Flow Blurring mechanism, i.e., turbulent hydrodynamic conditions are enforced in the inner mixing region, so that intense local vortices, eddies or kinematic fluctuations drive a strong mixing between the dope and the coagulating solutions. More precisely, said Flow Blurring mechanism guarantees that the quotient of random mean squared velocity of said local fluctuations over the axial velocity within the nebulizer is maximum and, consequently, the mixing efficiency is drastically increased to its maximum, while at the same time large local strain rates are exerted on the fibroin proteins in the inner mixing region and the exit region of the nebulizer. Common nebulizers available in the market are suitable for the implementation of the Flow Blurring conditions according to present invention.

Quantitatively, the above Flow Blurring conditions are achieved when the Reynolds number Re of the co-flow in the inner mixing region is sufficiently large. The Reynolds number Re is a dimensionless quantity correlated with the turbulence degree of a flow: the higher the Reynolds number Re, the more turbulent is said flow. Herein, the Reynolds number Re of the co-flow in the inner mixing region is given by Re = ρ_{c} vₐ L / η_{c}, wherein vₐ is the axial velocity of the co-flow in the inner mixing region, and L is the characteristic length of the transversal section of the inner mixing region. More precisely, based on mass and momentum conservation principles, the axial velocity vₐ is given herein by vₐ = (Q_{d} + Q_{c}) / A, wherein A is the transversal section area of the inner mixing region, Q_{d} is the flow rate of the dope stream in the first inlet section of the nebulizer, and Q_{c} is the flow rate of the co-flow in the second inlet section of the nebulizer.

In this context, when the co-flow is received at the second inlet section of the nebulizer at a flow rate Q_{c}, higher than the flow rate Q_{d} of the dope stream in the first inlet section, and the Reynolds number Re of the co-flow in the inner mixing region of the nebulizer is between 100 and 50000, then the mixing and residence times are of same order due to the high mixing efficiency of the turbulent flow.

Thus, within above ranges for the initial fibroin concentration, and for the co-flow Schmidt (Sc and Sc_{c}) and Reynolds (Re) numbers in the inner mixing region of the nebulizer, the aforementioned requirements are met in present invention, and turbulent motions generate a cascade of bifurcations in the mixing compound together with the fibres stretching, while water is extracted from said mixing compound and the pH value of the same mixing compound is modulated. As a result, a chaotic massive sequence of fibrous structures is generated in the inner mixing and discharge regions of the nebulizer, and a turbulent jet comprising a plurality of fibroin fibres exits the outlet section of said nebulizer.

In some particular embodiments, the second flow rate Q_{c} is higher than the first flow rate Q_{d}.

The magnitude of the mechanical stresses needed to create high-strength fibres and the efficiency of the solvent extraction are better.

In a preferred embodiment of the invention, the jet which exits through the outlet section of the nebulizer is introduced in a bath comprising an auxiliar coagulating solution, and said jet propagates through said bath, so that simultaneously the pH value of the mixing compound decreases, water is extracted from the mixing compound by said auxiliar coagulating solution, and a strain rate is exerted on the fibroin of the mixing compound by said auxiliar coagulating solution. In yet a further embodiment of the invention, the composition of the auxiliar coagulating solution is the same as the composition of the coagulating solution comprised in the co-flow. Although the turbulent character of the jet decays as it moves across the bath, during a finite time interval after ejection and if the auxiliar coagulating solution is an acid-alcohol mixture dissolved in water with a concentration an proportionalities within the same ranges as those of the coagulating solution comprised in the co-flow, but not necessarily the same values as those of said coagulating solution comprised in the co-flow, then there is a turbulent mixing of the mixing compound with the auxiliary coagulating solution, so that by means of the auxiliar coagulating solution simultaneously the pH value of said mixing compound is modulated, water is extracted from the same mixing compound, and a further strain rate is exerted on the fibroin of the mixing compound. As a result, the formation of fibroin fibres from the mixing compound is enhanced.

While the turbulent jet propagates through the bath, two fundamental outer actions, chemical and mechanical, are applied on the mixing compound along the turbulent process: the rate of water extraction and the local strain rate. Both actions compete to determine the final internal structure of the fibroin fibres.

When water extraction is faster, the structure becomes dominated by globular geometries whose size is fundamentally determined by the protein nature and concentration. In contrast, when the strain rate dominates, the water extraction merely responds to the local increase in surface area associated to the fibre stretching, and the fibre structure becomes nearly identical to that of the fibres naturally drawn by organisms. In between, both the chemical and mechanical rates homogeneously compete in the process to yield extensive characteristic repeating patterns along the fibres.

In yet a further preferred embodiment, the method further comprises the steps of situating at least one brush in the bath and in front of the outlet section of the nebulizer and rotating said brushes, so that the fibroin of the mixing compound comprised in the jet that exits the outlet section of the nebulizer is subject to an additional strain rate. The rotating brushes delay the decay of the turbulent character of the turbulent jet comprising the mixing compound, so that additional splits of the mixing compound into fibrous structures can take place. Moreover, at the same time, the inertia of the rotating motion of the brushes leads to the application of an additional stretching on the mixing compound, so that an additional strain rate is exerted in the fibroin of said mixing compound. As a result, the formation of fibroin fibres from the mixing compound is enhanced. In addition, the brushes act as a collecting device that drags and captures all the fibrous material generated, facilitating its management and keeping the bath cleaner for much longer times.

Interestingly, the generated fibroin fibres show a distribution of diameters that can span two or more orders of magnitude. In a preferred embodiment of the invention, the outlet section of the nebulizer has a circular transversal section with a diameter between 50 µm and 10 mm. As a result, the fibroin fibres of the turbulent jet have diameters of the order of tens of micrometres or smaller, so that said fibroin fibres are suitable for being used as scaffolds in Tissue Engineering.

In another preferred embodiment of the invention, the non-toxic carbon-based acid is acetic acid. The advantage of using acetic acid is that is a common, non-expensive and environmentally friendly acid.

In another preferred embodiment of the invention, the non-toxic hydrophilic alcohol is ethanol. Ethanol is a common, non-expensive and environmentally friendly alcohol with an outstanding capacity for bending proteins.

In a preferred embodiment of the invention, the solvent of the dope solution further comprises an auxiliary solute, so that the solubility of the fibroin in the dope solution is increased. An example of an auxiliar solute is CaCl₂.

Interestingly, the robust and resistant fibroin fibres generated according to present invention are particularly suitable to produce 100% biocompatible materials based on silk that may be used for several distinct uses.

Thus, another object of present invention refers to a biocompatible device comprising the plurality of fibroin fibres produced by a method according to any of the previous embodiments. The massive fibre production method developed herein leads to the creation of structures that are adequate for their usage as scaffolds in the context of Tissue Engineering, as base materials in biomedical composites, hypoallergenic fabrics or pads, as substrates in Controlled Drug Delivery, or as highly resistant raw material for fabrics and films under an appropriate post-processing.

### BRIEF DESCRIPTION OF DRAWINGS

To complete the description and in order to provide for a better understanding of the invention, a set of drawings is provided. Said drawings form an integral part of the description and illustrate several embodiments and data related to said embodiments, which should be not interpreted as restricting the scope of the invention, but just as examples of how the invention can be carried out. The accompanying drawings comprise specifically the following figures:
Fig. 1 shows a) a nebulizer surrounded by a bath, and b) the fibroin fibres generated with said nebulizer, according to a preferred embodiment of the invention.
Fig. 2 shows two Scanning Electron Microscope (SEM) images showing the appearance of the fibroin fibres obtained in the embodiment of Fig. 1a.
Fig. 3 shows an embodiment of the invention wherein the fibroin fibres that produced in a nebulizer surrounded by a bath, are collected with a rotational brush.
Fig. 4 shows the FTIR spectra of fibroin fibres produced in the embodiment of Fig. 3, aside from the lines corresponding to β-sheet and random coil and helix structures.
Fig. 5 shows six SEM images showing for six zoom-levels the appearance of the fibroin fibres obtained in the embodiment of Fig. 3.

### Numerical references used in the drawings:

| | |
|---|---|
| 1 | Nebulizer |
| 2 | Dope solution |
| 3 | Bath |
| 4 | Coagulating solution |
| 5 | Jet |
| 6 | Fibroin fibres |
| 7 | Brush |

### DETAILED DESCRIPTION OF THE INVENTION

In this section, several non-limitative embodiments of the invention are provided. We remark that the invention relates to a method for the massive production of fibroin fibres from a dope solution by subjecting said dope solution to turbulent hydrodynamic conditions.

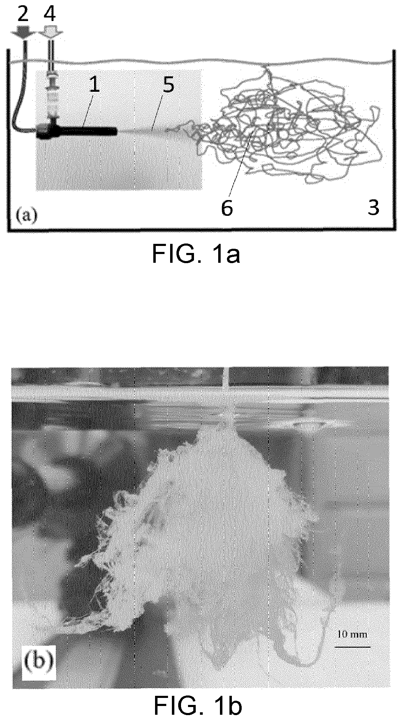

Fig. 1a shows an embodiment of the invention wherein a nebulizer 1 is configured to implement the Flow Blurring conditions in a dope solution 2, the nebulizer 1 being surrounded by a bath 3 comprising a 10 L 1 M solution, wherein the solvent is ultrapure water, and the solute comprises a mixture of 20% acetic acid and 80% ethanol. In particular, herein it is used the "OneNeb" Flow Blurring nebulizer, from "Ingeniatrics Tec. S.L." (Spain). Said nebulizer 1 comprises a central nebulizer, the nebulizer having a first inlet section, a second inlet section, a cylindrical inner mixing region arranged to receive a first flow from the first inlet section and a second flow from the second inlet section, and an circular outlet section with the same diameter as the transversal section of the inner mixing region. In particular, herein the diameter D of both the transversal section of the inner mixing region and of the outlet section is equal to 240 µm.

The nebulizer 1 is further configured to receive (e.g., with a valve) a dope stream in the first inlet section of the nebulizer at a first flow rate Q_{d}, the dope stream comprising a dope solution 2. Herein, the first flow rate Q_{d} of the dope stream is equal to 2.2 ml/min, and the dope solution 3 is a mixture comprising fibroin dissolved in a 1 M solution of CaCl₂ in ultrapure water. In particular, the fibroin used herein is *Bombyx Mori* silk fibroin, although other types of fibroins can be used equivalently. The initial concentration of the fibroin in the dope solution 2 is 29% wt, i.e., close to that found in Nature.

In natural processes, flow rates between 0.1 and 2.6 µl/min are typically found, i.e., between 770 and 5000 times smaller than those used in this embodiment of the invention, in accordance to a diameter of both the transversal section of the inner mixing region and of the outlet section of 240 µm. Such flow rates may be, however, even 10 to 1000 times higher in different embodiments, corresponding to diameters of 1 mm or more. This would imply a much higher efficiency in the creation of the biocompatible fibres.

Furthermore, the nebulizer 1 is configured to receive (e.g., with a valve) a co-flow in the second inlet section of the nebulizer at a second flow rate Q_{c}, the co-flow comprising a 1 M coagulating solution 4 with a pH value of 4, wherein the solvent is ultrapure water, and the solute comprises a mixture of 20% acetic acid and 80% ethanol. Herein, the second flow rate Q_{c} of the co-flow in the second inlet section is equal to 50 ml/min.

In addition, the central nebulizer of the nebulizer 1 can be configured to pre-mix the dope stream comprising the dope solution 2 and the co-flow comprising the coagulating solution 4 in its inner mixing region. As a result, a two-phase mixing compound comprising the dope and the coagulating co-flow is produced.

In this context, since the viscosity η_{c} of the coagulating solution 4 is equal to approximately 1.2 mPa·s, the density ρ_{c} of the same coagulating solution 4 is equal to 800 kg/m³, and D_{c} is mass diffusivity of water in the coagulating solution 4, approximately equal to 2.7×10⁻⁹ m²/s, the respective Schmidt number Sc_{c} of the co-flow in the inner mixing region is approximately equal to 0.0018.

On the other hand, the Reynold number Re of the co-flow in the inner mixing region is determined by the above values of the viscosity η_{c} and the density ρ_{c} of the coagulating solution 4, as well as by the diameter D of the transversal section of the inner mixing region, and by the axial velocity vₐ of the mixing compound in said inner mixing region, wherein here the axial velocity vₐ is given by vₐ = (Q_{d} + Q_{c}) / (4 π D²). More precisely, the Reynolds number Re is given by Re = ρ_{c} vₐ D / η_{c} = ρ_{c} (Q_{d} + Q_{c}) / ( 4 π η_{c} D), so that herein the Reynolds number Re of the co-flow in the inner mixing region of the nebulizer is equal to 190, that is, said co-flow is turbulent in the inner mixing region.

Hence, the above vales of the initial concentration of the fibroin in the dope solution 2, and of the Schmidt Sc and Reynolds Re numbers of the co-flow in the inner mixing region of the nebulizer, are such that the pH value of the mixing compound is modulated and water is extracted from said mixing compound, while at the same time the mixing compound is stretched by said co-flow and the turbulent motions generate a cascade of bifurcations of the fibroin comprised in said mixing compound. As a result, the mixing compound splits in the inner mixing region of the nebulizer into a sequence of numerous fibrous structures, and a turbulent jet 5 exits through the outlet section of the nozzle, wherein said turbulent jet 5 comprises a sequence of numerous fibroin fibres 6. The nebulizer 1 is configured to exit said jet 5, so that the jet 5 is introduced to the bath 3.

The turbulent character of the jet 5 decays while it propagates through the bath 3. Along the axial, turbulent decay evolution of the jet 5, the mixing compound is exposed to characteristic spectra of flow patterns (highly extensional regions, instantaneous stagnation points, branching eddies) and turbulent intensities of decaying jets. The final fibroin fibres 6 are assumed to have a uniform chemical composition (i.e., the final mass concentration of fibroin is close to 100%). This concentration obviously changes along the process due to water extraction by diffusion across the bath 3, but the mass of fibroin is conserved along the process involving stretching, folding, and splitting of the fibrous structure.

Fig. 1b shows the cotton-like shape of the fibroin fibres 6 collected from a single 25 second ejection of 1.5 g of the dope solution 2 in the embodiment shown in Fig. 1a. Herein, due to the relatively large size of the bath 3 (i.e., 10 L) compared to the mass of the ejected dope solution 2 ejected (1.5 g), the composition of the bath 3 is assumed constant.

While the turbulent jet 5 propagates through the bath 3, two fundamental outer actions, chemical and mechanical, are applied on the mixing compound along the turbulent process: the rate of water extraction and the local strain rate. Both actions compete to determine the final internal structure of the fibroin fibres 6. In this context, when water extraction is faster, a pattern of globular geometries appears along the fibroin fibres 6 whose size mainly depends on the protein nature and concentration. In contrast, when the strain rate dominates, the water extraction merely responds to the local increase in surface area associated to the fibre stretching, and the fibre structure approaches that of the silk fibres naturally drawn by organisms. In between, both the chemical and mechanical rates homogeneously compete in the process to yield extensive characteristic repeating patterns along the fibroin fibres 6. This competition only occurs if the fibroin concentration is sufficiently high for water extraction to be controlled by mechanical actions, at least over a significant part of the spectrum of the co-flow characteristic turbulent scales. Said scales may be any scale comprised between the diameter of the outlet section of the nebulizer and the dimension of the device used to collect the formed fibres from the bath (in the present case, the brush).

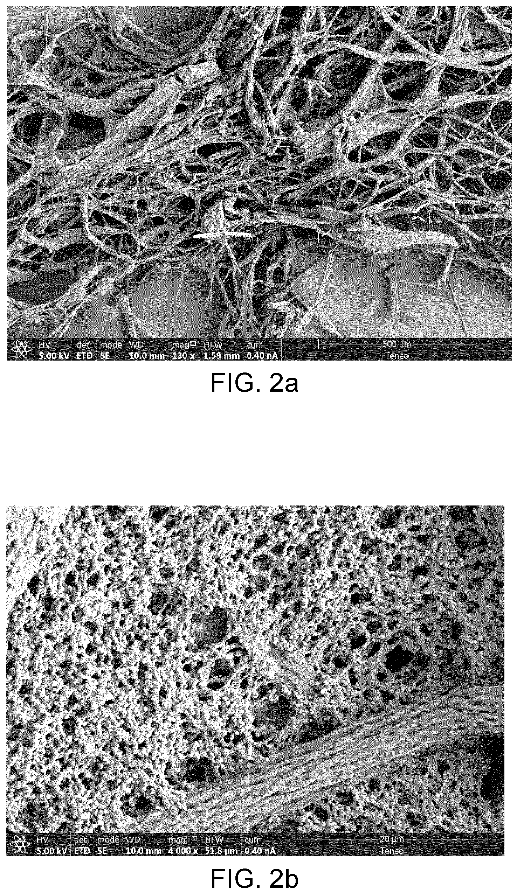

Fig. 2 shows two Scanning Electron Microscope (SEM) images showing the appearance of fibroin fibres 6 obtained in the embodiment shown in Fig. 1a. On one hand, Fig. 2a shows a millimeter-scale appearance of the fibroin fibre 6. The absence of any additional mechanical input leaves a structure dominated by the inertia of the large scales, i.e., turbulent scales. On the other hand, Fig. 2b shows an illustration wherein the aforementioned non-stretched globular structures are readily apparent, together with a mildly stretched fibre that exhibits a characteristic micro-structure.

The drawing action of the embodiment shown in Fig. 1a can be enhanced by the application of a mechanical input on the turbulent jet 5, after said jet 5 exits the outlet section of the nebulizer.

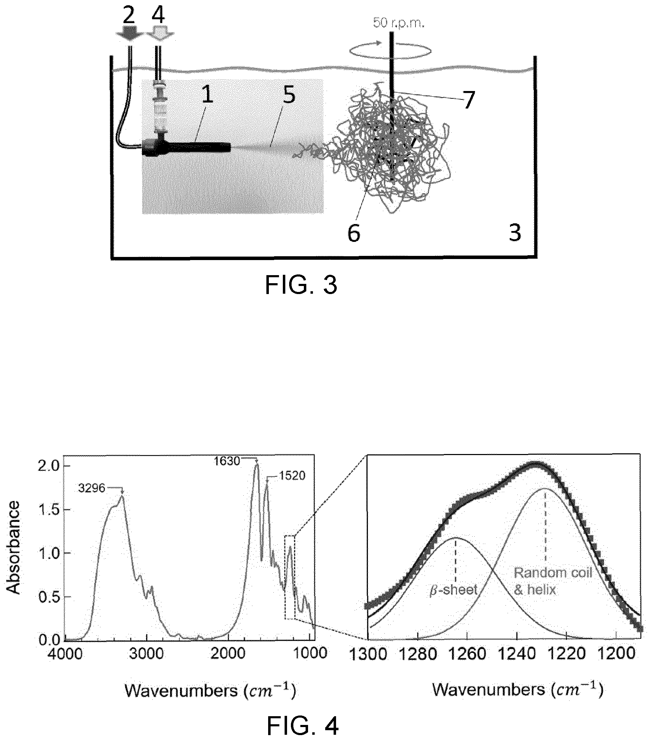

Fig. 3 shows a preferred embodiment of the invention wherein the drawing action is enhanced by collecting the cotton-like shape fibroin fibres 6 with a rotational brush 7. Herein, said brush 7 rotates at 50 r.p.m. Concerning the nebulizer 1 of this preferred embodiment, it is exactly the same as the one of Fig. 1a, as it is also the case of the bath 3. Regarding the dope solution 2 and the coagulating solution 4, in this embodiment of Fig. 3 same flow rates and initial compositions of said solutions as the embodiment of Fig. 1a are used.

Advantageously, in the embodiment of Fig. 3, the wide spectrum of scales and the inertia (herein, mainly that generated by the co-flow and that related to the rotating motion induced by the brush 7) of the large ones guarantee that fibres with the smaller diameters undergo high and relatively homogeneous stresses along their span, i.e., along the propagation of the turbulent jet 5 through the bath 3. Indeed, those stresses can overcome by orders of magnitude the applied macroscopic pressure that drives the combined flow of the dope stream and the co-flow in the nebulizer 1, mostly because the ratio of the former over the latter is small (herein, about 0.04).

Furthermore, the signatures of the fibroin fibres 6 produced in the embodiment of Fig. 3 approach or are nearly identical to those signatures of fibres produced by spinning organisms.

Fig. 4 shows the Fourier-transform infrared spectroscopy (FTIR) spectrum of fibroin fibres 6 produced in the embodiment of Fig. 3, with the lines corresponding to β-sheet and random coil and helix structures. FTIR is a well-established analytical technique used to identify organic and polymeric materials and uses infrared light to scan test samples and observe chemical properties. Since each molecule in the fibroin fibres 6 absorbs infrared radiation at a characteristic frequency, all molecules can be identified by their characteristic absorption spectrum. The FTIR instrument sends infrared radiation of approximately 10000 to 100 *cm*⁻¹ through the fibroin fibres. Part of such radiation is absorbed by the fibres and is converted into rotational or vibrational energy by the molecules of the fibroin.

The resulting signal at the detector of the instrument presents the spectrum of Fig. 4, which represents a molecular fingerprint of the fibroin fibres 6 produced with the embodiment of Fig. 3. In Fig. 4, the total spectrum generated is thus a series function of absorbed energy response. A particular peak of energy at a certain wavenumber is the result of certain chemical and matrix factors in the fibroin fibres 6, and the peak height is directly proportional to concentration of a particular structure present in the fibroin fibres 6. The deconvoluted spectrum in the range 1180 - 1330 *cm*⁻¹ shows characteristic fingerprint peaks of β-sheet (-1265 *cm*⁻¹) and random coil and helix (-1230 *cm*⁻¹) structures according to earlier literature reports (Fang et al. (2016), Insights into silk formation process: correlation of mechanical properties and structural evolution during artificial spinning of silk fibres. ACS Biomaterials Science & Engineering, 2(11), 1992-2000.)

Thus, the resulting microscopic fibrous structure in the embodiment of Fig. 3 exhibits clearly identifiable fibroin fibres 6 with a relatively constant diameter (typical deviations below 20% along the fibre length), especially those with diameters below 1 µm. Each individual fibre can be defined by a set of diameter measurements along its axis, and a set of points defining its axis from one end to the other which are normally determined by bifurcations. In addition, some fibres present a flattened cross section, especially those with apparent diameters beyond 10-20 µm.

A simplified yet sufficiently descriptive and quantitative approach has been chosen for characterizing the silk fibres 6 obtained in the process to evaluate the performance of the method as follows:
- Each fibre is defined by a diameter d, defined as the average diameter along said fibre, and four points along the fibre axis: two central points and two ends.
- The length I of the fibre is computed as the sum of the three lengths of the segments defined by the four axial points.
- The position of the central points is chosen at the two locations where the curvature is maximum, and both points are separated at least 5 diameters, except in cases where the fibre slenderness (i.e., the ratio between its length I and diameter d, I/d) is below 20. When the fibre is straight, each central point is set equidistant from the others.
- In relatively frequent cases, the fibre ends beyond the frame limits. In cases where two or more alternative SEM micrographs with different zooming have been taken, and the fibre can be identified in all of them, the axial points are determined on the outmost zoom picture. However, this is not possible in most cases, and consequently the fibre length I statistics is less reliable at the large side of the size spectrum.

Using the above procedure, 620 fibroin fibres 6 were identified in the embodiment of Fig. 3 from 17 SEM images. In said 620 fibroin fibres 6 the fibre diameters d ranged from around 17 nm to around 4 µm, the average of all diameters (d) was equal to 413 nm, and the average length (I) resulted around 10 µm.

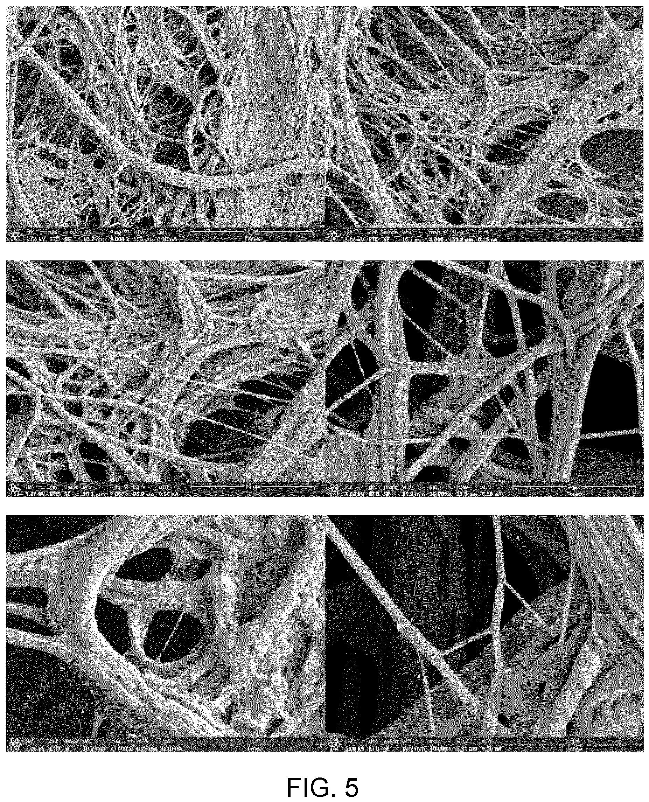

Fig. 5 shows six SEM images for six zoom-levels, among the 17 SEM images used to identify the 620 fibroin fibres obtained in the embodiment of Fig. 3.

## Claims

1. Method for generating a plurality of fibroin fibres,
the method being **characterized in that** it comprises the steps of:
providing a nebulizer, the nebulizer having a first inlet section, a second inlet section, an inner mixing region arranged to receive a first flow from the first inlet section and a second flow from the second inlet section, and an outlet section;
receiving a dope stream in the first inlet section of the nebulizer at a first flow rate (Q_{d}), the dope stream comprising a dope solution (2) of an initial concentration of fibroin in a solvent which comprises water;
receiving a co-flow in the second inlet section of the nebulizer at a second flow rate (Q_{c}), the co-flow comprising a coagulating solution (4) wherein the solvent is water, and the solute comprises a mixture of a non-toxic carbon-based acid and a non-toxic hydrophilic alcohol, and wherein the pH value of said coagulating solution (4) is between 2 and 6;
mixing the dope stream and the co-flow in the inner mixing region of the nebulizer, producing a mixing compound so that simultaneously the pH value of the mixing compound decreases with respect to the pH value of the dope solution (2) by the action of the non-toxic carbon-base acid comprised in the co-flow, water is extracted by the non-toxic hydrophilic alcohol comprised in the co-flow, as long as the concentration of water in the mixing compound is lower than the concentration of water in the dope solution (2), and a strain rate is exerted on the fibroin of the mixing compound by the co-flow; and
exiting through the outlet section of the nebulizer a jet (5) comprising the mixing compound;
and wherein the initial concentration of fibroin in the dope solution (2) is comprised between 5% wt and 50% wt, the Schmidt number (Sc) of the water in the dope, is between 1 and 10000, and the Reynolds number (Re) of the co-flow in the inner mixing region of the nebulizer is between 100 and 50000, so that the mixing compound splits in the inner mixing region of the nebulizer into a sequence of fibrous structures and the jet (5) that exits the outlet section of the nebulizer is turbulent and comprises a plurality of fibroin fibres (6).

2. Method according to claim 1, wherein the jet (5) which exits through the outlet section of the nebulizer is introduced in a bath (3) comprising an auxiliar coagulating solution, and said jet (5) propagates through said bath (3), so that simultaneously the pH value of the mixing compound decreases, water is extracted from the mixing compound by said auxiliar coagulating solution, and a strain rate is exerted on the fibroin of the mixing compound by said auxiliar coagulating solution.

3. Method according to claim 2, wherein the composition of the auxiliar coagulating solution is the same as the composition of the coagulating solution (4) comprised in the co-flow.

4. Method according to claim 3, wherein the method further comprises the steps of situating one or more brushes (7) in the bath (3) and in front of the outlet section of the nebulizer and rotating said brushes (7), so that the fibroin of the mixing compound comprised in the jet (5) that exits the outlet section of the nebulizer is subjected to an additional strain rate.

5. Method according to any of the preceding claims, wherein the second flow rate Q_{c} is higher than the first flow rate Q_{d}.

6. Method according to any of the preceding claims, wherein the outlet section of the nebulizer has a circular transversal section with a diameter between 50 µm and 10 mm.

7. Method according to any of the preceding claims, wherein the non-toxic carbon-based acid is acetic acid.

8. Method according to any of the preceding claims, wherein the non-toxic hydrophilic alcohol is ethanol.

9. Method according to any of the preceding claims, wherein the solvent of the dope solution (2) further comprises an auxiliar solute, so that the solubility of the fibroin in the dope solution (2) is increased.

10. Biocompatible device comprising the plurality of fibroin fibres (6) produced by a method according to any of claims 1-9.
